# EUROPEAN PATENT SPECIFICATION

(11) **EP 3 902 536 B1**
(45) Date of publication and mention of the grant of the patent: **03.09.2025**
(21) Application number: 19903186.5
(22) Date of filing: 26.12.2019
(51) Int. Cl.: A61K 31/196, A61K 31/216, A61K 31/606, A61K 9/00, A61K 47/26, A61K 47/38, A61K 47/12, A61K 47/20, A61K 47/36, A61K 47/42, A23L 33/10, A61P 25/28, A61P 25/24, A23K 20/111, A23K 50/40, A61K 9/48

(54) **COMPOSITIONS COMPRISING 5-BENZYLAMINOSALICYLIC ACID COMPOUNDS FOR USE IN TREATING NEURODEGENERATIVE DISORDERS IN A COMPANION ANIMAL**
ZUSAMMENSETZUNGEN, DIE 5-BENZYLAMINOSALICYLSÄURE-VERBINDUNGEN ENTHALTEN, ZUR VERWENDUNG BEI DER BEHANDLUNG VON NEURODEGENERATIVEN STÖRUNGEN BEI HAUSTIEREN
COMPOSITIONS COMPRENANT DES COMPOSÉS DE L'ACIDE 5-BENZYLAMINOSALICYLIQUE POUR UTILISATION DANS LE TRAITEMENT DE TROUBLES NEURODÉGÉNÉRATIFS CHEZ UN ANIMAL DE COMPAGNIE

(30) Priority: 28.12.2018 US 201862785903 P
(43) Date of publication of application: 03.11.2021
(73) Proprietor: GNT Pharma Co., Ltd, Yongin-si, Gyeonggi-do 17096 (KR)
(72) Inventor: LEE, Jinhwan, Yongin-si 168NN-172NN (KR); MOON, Jae Bong, Seongnam-si 131NN-137NN (KR); GWAG, Byoung Joo, Yongin-si 168NN-172NN (KR)
(74) Representative: HGF
(86) International application number: PCT/IB2019/001409
(87) International publication number: WO 2020/136441

(56) References cited:
- EP-A2- 2 394 645
- WO-A1-2004/000786
- WO-A1-86/03199
- WO-A1-94/13663
- WO-A2-2011/081445
- KR-A- 20030 058 934
- US-A1- 2007 298 129
- US-A1- 2007 298 129
- BAEK IN-SUN ET AL: "AAD-2004 Attenuates Progressive Neuronal Loss in the Brain of Tg-betaCTF99/B6 Mouse Model of Alzheimer Disease", EXPERIMENTAL NEUROBIOLOGY, vol. 22, no. 1, 30 March 2013 (2013-03-30), pages 31 - 37, XP055951509, ISSN: 1226-2560, DOI: 10.5607/en.2013.22.1.31
- NEUS BOSCH MARIA ET AL: "Dogs with Cognitive Dysfunction Syndrome: A Natural Model of Alzheimer's Disease", CURRENT ALZHEIMER RESEARCH, 1 January 2012 (2012-01-01), pages 298 - 314, XP055951624, Retrieved from the Internet <URL:https://pubmed.ncbi.nlm.nih.gov/21875411/> [retrieved on 20220815]
- SHEFFY B. E. ET AL: "Nutrition and metabolism of the geriatric dog.", THE CORNELL VETERINARIAN, 1 April 1985 (1985-04-01), pages 325 - 325, XP055951570, Retrieved from the Internet <URL:pubmed:2985334> [retrieved on 20220815]
- ANONYMOUS: "SUMMARY OF PRODUCT CHARACTERISTICS Reconcile", 13 July 2018 (2018-07-13), pages 1 - 19, XP055951582, Retrieved from the Internet <URL:chrome-extension://efaidnbmnnnibpcajpcglclefindmkaj/https://www.ema.europa.eu/en/documents/product-information/reconcile-epar-product-information_en.pdf> [retrieved on 20220815]
- ANONYMOUS: "Crisdesalazine", COGNITIVE VITALITY REPORTS, 18 August 2020 (2020-08-18), pages 1 - 10, XP055951505, Retrieved from the Internet <URL:chrome-extension://efaidnbmnnnibpcajpcglclefindmkaj/https://www.alzdiscovery.org/uploads/cognitive_vitality_media/Crisdesalazine.pdf> [retrieved on 20220815]
- CHAPAGAIN DURGA ET AL: "Cognitive Aging in Dogs", GERONTOLOGY, vol. 64, no. 2, 25 October 2017 (2017-10-25), CH, pages 165 - 171, XP093086652, ISSN: 0304-324X, Retrieved from the Internet <URL:https://karger.com/ger/article-pdf/64/2/165/2838777/000481621.pdf> DOI: 10.1159/000481621

## Description

### RELATED APPLICATIONS

This application claims the benefit of and priority to U.S. Provisional Patent Application serial number 62/785,903, filed December 28, 2018.

### FIELD OF THE DISCLOSURE

The present disclosure relates to pharmaceutical compositions for use in treating cognitive dysfunction syndrome (CDS) in aging companion animals, comprising a 5-benzylaminosalicylic acid compound of formula (I), or a pharmaceutically acceptable salt thereof, and a pharmaceutically acceptable excipient.

### BACKGROUND

The number of companion animals worldwide is steadily increasing. The number of household dogs and cats in top 20 countries is estimated to be approximately 256 million and approximately 236 million, respectively. Household dogs and cats live much longer these days, due to improvement in nutrition, disease treatment, and outstanding veterinary and pet-owner care. Therefore, there is growing interest in the treatment of age-related diseases in companion animals.

A number of aged or aging dogs and cats suffer from cognitive impairment that results from nervous system disorders including CDS, dysthymia, involutive depression, and confusional syndrome. Cognitive impairment is defined as trouble in remembering, learning new things, concentrating, or making decisions that can affect daily life. CDS is an age-related degenerative brain disease in dogs and cats. The prevalence of CDS has been reported to range from 14% to 60% in dogs over 8 years old [1-4]. Other reports show that 62% of companion dogs at 11-16 years old suffer from CDS, with the prevalence increasing markedly with age [5]. Therefore, a large number of dogs are afflicted with CDS, which can worsen human-animal bond, lower the quality of life in animals, and consequently shorten the life span of the animals [6-8].

Pathophysiological changes seen in canine CDS include cerebrocortical and basal ganglia atrophy; increase in ventricle size; demyelination; an increase in the size and number of glial cells; neuronal loss especially in the cortical regions over the hippocampus; axonal degeneration; an accumulation of beta-amyloid plaques [6, 9, 10]. Cats with CDS are also accompanied by decreased number of neurons, beta amyloid deposition, and increased glial cells similar to canine CDS. In contrast to Alzheimer's disease (AD) in humans, neurofibrillary tangles widespread in AD have not been identified in the brains of dogs and cats with CDS, suggesting that CDS in dogs and cats is distinguishable from AD [11].

Dogs and cats diagnosed with CDS exhibit progressive impairment in cognitive and neurological functions. They show various behavioral problems such as getting lost in house, showing less daytime activity, no longer greeting to their families or other familiar animals, no reaction when called by name, going around in a circle or urination in unusual region [10, 12-15].

Although the rapidly growing population of dogs and cats with CDS is recently observed, CDS remains an unmet medical need. Selegiline, a selective irreversible inhibitor of monoamine oxidase B (MAO-B), is the only pharmaceutical medication that is approved by FDA for treating CDS [16]. Previous reports showed that selegiline enhanced cognitive function and improved behavioral function. However, recent data indicate that its beneficial effects are not maintained for a long time, indicating that selegiline therapy is just a symptomatological treatment. For example, the cognitive function in CDS dogs were improved within the first 2 weeks after selegiline treatment, but the beneficial effect in most of the dogs disappeared at 8 weeks after treatment [17-19]. Moreover, a large clinical trial involving 474 dogs with CDS over the age of 8 years old concluded that selegiline treatment should be administered at early onset of clinical signs [19].

A number of non-pharmacological therapies have been commercially available including dietary supplementation with antioxidants, L-carnitine, and omega-3 fatty acids that is just to improve the welfare of the dog by relieving the anxiety and supporting cognitive function. For optimal results, such dietary supplement should begin at the early stage of canine CDS. As there is no cure for CDS in canine and feline, disease-modifying compounds and methods that can halt or slow down the progression of CDS need to be developed.

Over 400 clinical trials for the treatment of AD were conducted between 2002 and 2012, but memantine, a low to moderate affinity N-methyl-D-aspartate receptor antagonist, was the only drug approved for symptomatic treatment of AD based upon findings that it showed a small improvement in mental function and ability to perform daily activities in moderate-to-severe AD. Since then, large phase III clinical trials of drugs targeting beta amyloid, inflammation, or oxidative stress have been conducted for AD but all failed in showing beneficial effects, making the situation worse. However, nearly all the failed drugs reduced amyloid plaque burden and improved cognitive function in the two standard mouse models of AD, Tg2576 and APP/PS1 that overexpress familial AD mutations of genes, mutant amyloid precursor protein (APP), or APP and presenilin, respectively. Thus, pharmacological efficacy of drug candidates proven in preclinical animal models of AD is not translated for the treatment of AD in humans.
US2007/298129 A1 describes compounds and compositions for treating neuronal death or neurological dysfunction,; and In-Sun Baek et al., Exp Neurobiol. 2013 Mar 31;22(1):31-37 considers that AAD-2004 attenuates progressive neuronal loss in the brain of Tg-betaCTF99/B6 mouse model of Alzheimer Disease; Neus Bosch et al., Curr Alzheimer Res. 2012 Mar;9(3):298-314 considers appropriate models for Alzheimer's disease (AD) involving animals.

### DETAILED DESCRIPTION

A 5-benzylaminosalicylic acid compound, or a pharmaceutically acceptable salt thereof, has been used for the treatment of AD and neurodegenerative diseases (US. Pat. No. 6,964,982). In a previous study, 2-hydroxy-5-[2-(4-trifluoromethylphenyl)ethylamino]benzoic acid is verified as a potent spin-trapping molecule and microsomal prostaglandin E(2) synthase-1 (mPGES-1) inhibitor effective at nanomolar concentrations, which results in not only blockade of neuronal death, axonopathy, and autophagosome formation but also increases of motor function activity and life span in a mouse model of amyotrophic lateral sclerosis [20].

A 5-benzylaminosalicylic acid compound, or a pharmaceutically acceptable salt thereof, was explored for the treatment of CDS in companion animals. Surprisingly, oral administration of 2-hydroxy-5-[2-(4-trifluoromethyl-phenyl)ethylamino]benzoic acid for longer than 4 weeks markedly improved cognitive and neurobehavioral functions in aged companion dogs suffering from severe CDS. Moreover, the beneficial effects of 2-hydroxy-5-[2-(4-trifluoromethyl-phenyl)ethylamino]benzoic acid were maintained for at least 4 weeks after the last administration. It was thus suggested that 2-hydroxy-5-[2-(4-trifluoromethylphenyl)ethylamino]benzoic acid could be a potential therapeutic option for treating CDS through inhibiting both oxidative stress and inflammation.

### Technical Problem

Accordingly, the present disclosure provides pharmaceutical compositions for use in treating CDS in a companion animal.

### Technical Solution

The present invention provides a composition comprising a compound of formula (I) for use in treating cognitive dysfunction syndrome (CDS) in a companion animal: 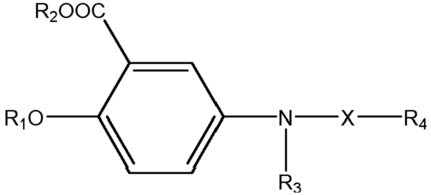 wherein,
X is selected from CO, SO₂ and (CH₂)*ₙ*;
R₁ is selected from hydrogen, C₁-C₆ alkyl and C₁-C₆ alkanoyl;
R₂ is selected from hydrogen and C₁-C₆ alkyl;
R₃ is selected from hydrogen and a C₁-C₅ acetyl group; and
R₄ is a phenyl group which is unsubstituted or substituted with one or more substituents selected from the group consisting of nitro, halogen, C₁-C₆ alkyl, C₁-C₆ haloalkyl, C₁-C₅ alkoxy, and C₁-C₅ haloalkoxy;
n is an integer from 1 to 5, inclusive;
or a pharmaceutically acceptable salt thereof, and a pharmaceutically acceptable excipient.

In some embodiments, a number of compounds of formula (I) have been prepared and evaluated. In some embodiments, the compositions comprise a 5-benzylaminosalicylic acid compound of formula (I) or its pharmaceutically acceptable salt.

In some embodiments, the 5-benzylaminosalicylic acid compound is 5-benzylaminosalicylic acid itself.

Preferable examples of 5-benzylaminosalicylic acid compounds include, but are not limited to, 2-hydroxy-5-phenethylamino-benzoic acid (Compound 1), 2-hydroxy-5-[2-(4-trifluoromethyl-phenyl)-ethylamino]-benzoic acid (Compound 2), 2-hydroxy-5-[2-(3-trifluoromethyl-phenyl)-ethylamino]-benzoic acid (Compound 3), 5-[2-(3,5-bis-trifluoromethyl-phenyl)-ethylamino]-2-hydroxy-benzoic acid (Compound 4), 2-hydroxy-5-[2-(2-nitro-phenyl)-ethylamino]-benzoic acid (Compound 5), 5-[2-(4-chloro-phenyl)-ethylamino]-2-hydroxy-benzoic acid (Compound 6), 5-[2-(3,4-difluoro-phenyl)-ethylamino]-2-hydroxy-benzoic acid (Compound 7), 5-[2-(3,4-dichloro-phenyl)-ethylamino]-2-hydroxy-benzoic acid (Compound 8), 5-[2-(4-fluoro-2-trifluoromethylphenyl)-ethylamino]-2-hydroxy-benzoic acid (Compound 9), 5-[2-(2-fluoro-4-trifluoromethyl-phenyl)-ethylamino]-2-hydroxy-benzoic acid (Compound 10), 2-hydroxy-5-[2-(4-methoxy-phenyl)-ethylamino]-benzoic acid (Compound 11), 2-hydroxy-5-(2-o-tolyl-ethylamino)-benzoic acid (Compound 12), 2-hydroxy-5-(3-phenyl-propylamino)-benzoic acid (Compound 13), 2-hydroxy-5-[3-(4-trifluoromethyl-phenyl)-propylamino]-benzoic acid (Compound 14), 5-[3-(4-fluoro-phenyl)-propylamino]-2-hydroxy-benzoic acid (Compound 15), 5-[3-(3,4-dichloro-phenyl)-propylamino]-2-hydroxy-benzoic acid (Compound 16), 2-hydroxy-5-(3-p-tolyl-propylamino)-benzoic acid (Compound l7), 2-acetoxy-5-[2-(4-trifluoromethyl-phenyl)-ethylamino]-benzoic acid (Compound 18), 5-[2-(2-chloro-phenyl)-ethylamino]-2-hydroxy-benzoic acid (Compound 19), 5-benzylaminosalicylic acid (Compound 20), 5-(4-nitrobenzyl)aminosalicylic acid (Compound 21), 5-(4-chlorobenzyl)aminosalicylic acid (Compound 22), 5-(4-trifluoromethylbenzyl)aminosalicylic acid (Compound 23), 5-(4-fluorobenzyl)aminosalicylic acid (Compound 24), 5-(4-methoxybenzyl)aminosalicylic acid (Compound 25), 5-(2,3,4,5,6-pentafluorobenzyl)aminosalicylic acid (Compound 26), 5-(4-nitrobenzyl)amino-2-hydroxy ethylbenzoate (Compound 27), 5-(4-nitrobenzyl)-N-acetylamino-2-hydroxy ethylbenzoate (Compound 28), 5-(4-nitrobenzyl)-N-acetylamino-2-acetoxy ethylbenzoate (Compound 29), 5-(4-nitrobenzoyl)aminosalicylic acid (Compound 30), 5-(4-nitrobenzenesulfonyl)aminosalicylic acid (Compound 31), 5-[2-(4-nitrophenyl)-ethyl]aminosalicylic acid (Compound 32), and 5-[3-(4-nitro-phenyl)-n-propyl]aminosalicylic acid (Compound 33). In certain preferred embodiments, the compound of formula (I) is Compound 2, 2-hydroxy-5-[2-(4-trifluoromethylphenyl)ethylamino]benzoic acid or a pharmaceutically acceptable salt thereof. In some embodiments, the compound of formula (I) has the structure

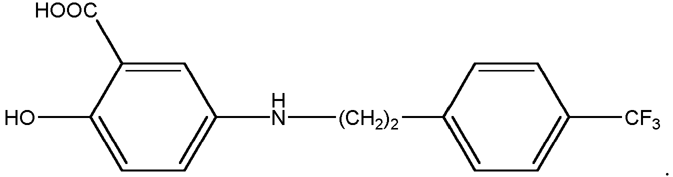

The 5-benzylaminosalicylic acid compound or its pharmaceutically acceptable salt of the present disclosure can be prepared by, but is not limited to, the reaction schemes represented in US. Pat. No. 6,573,402.

The compounds of formula (I) treat CDS in a companion animal. In some embodiments, the treatment is through concurrent pharmacological inhibition of oxidative stress and inflammation. In some embodiments, the treatment is through inhibiting oxidative stress and prostaglandin E₂ synthesis. In some embodiments, the treatment is through inhibiting oxidative stress and microsomal prostaglandin E synthase-1.

In some embodiments, the companion animal exhibits symptoms involving behavioral changes selected from appetite, drinking behavior, vocalization, elimination behavior, sleeping pattern, aimless behavior, adaptive capabilities, social behavior, perceptual ability, disorientation, and memory. In some embodiments, the companion animal exhibits symptoms involving behavioral changes selected from vocalization, elimination behavior, sleeping pattern, aimless behavior, social behavior, perceptual ability, disorientation, and memory. In some embodiments, the companion animal exhibits symptoms involving behavioral changes selected from sleeping pattern, social behavior, disorientation, and memory. In some embodiments, the companion animal exhibits symptoms involving changes in memory. In some embodiments, the companion animal exhibits symptoms involving behavioral changes selected from orientation (e.g., staring blankly and getting lost in the home), memory (e.g., lack of recognition of owners and house-soiling), apathy (e.g., reduced time spent active and avoiding contact with owners), impaired olfaction (e.g., difficulty finding food), and locomotion.

In some embodiments, the companion animal exhibits symptoms involving behavioral changes selected from spatial orientation, social interaction, sleep-wake cycle, and house soiling.

In some embodiments, the companion animal exhibits pathophysiological changes. In some embodiments, the pathophysiological changes are selected from cerebrocortical atrophy; basal ganglia atrophy; increase in ventricle size; demyelination; an increase in the size of glial cells; an increase in the number of glial cells; neuronal loss especially in the cortical regions over the hippocampus; axonal degeneration; and an accumulation of beta-amyloid plaques. In some embodiments, the pathophysiological changes are selected from an increase in the size of glial cells; an increase in the number of glial cells; neuronal loss; and an increase in beta-amyloid deposition.

In some embodiments, the companion animal is selected from a cat, a chinchilla, a dog, a ferret, a gerbil, a guinea pig, a hamster, a hedgehog, a mouse, a rabbit, and a rat. In certain preferred embodiments, the companion animal is a cat or a dog. In some embodiments, the companion animal is a canine or a feline.

### Definitions

The definitions for the terms described below are applicable to the use of the term by itself or in combination with another term.

The term "acetoxy" refers to a group represented by the general formula hydrocarbylC(O)O-, preferably alkylC(O)O-.

The term "acetyl" refers to a group represented by the general formula CH₃C(O)-.

An "alkyl" group (including 'alkyl' of haloalkyl) or "alkane" is a straight chained or branched non-aromatic hydrocarbon which is completely saturated. Typically, a straight chained or branched alkyl group has from 1 to about 20 carbon atoms, preferably from 1 to about 10 unless otherwise defined. A C₁-C₆ straight chained or branched alkyl group is also referred to as a "lower alkyl" group. In some embodiments, the alkyl is C₁-C₅ alkyl, and more preferably C₁-C₃ alkyl. More specifically, preferable alkyl groups include, but are not limited to, methyl, ethyl, propyl, isopropyl, butyl, sec-butyl and tert-butyl.

Moreover, the term "alkyl" (or "lower alkyl") as used throughout the specification, examples, and claims is intended to include both "unsubstituted alkyls" and "substituted alkyls", the latter of which refers to alkyl moieties having substituents replacing a hydrogen on one or more carbons of the hydrocarbon backbone. Such substituents, if not otherwise specified, can include, for example, a halogen, a hydroxyl, a carbonyl (such as a carboxyl, an alkoxycarbonyl, a formyl, or an acyl such as an alkylC(O)), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a silyl ether, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain can themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthiols, carbonyls (including ketones, aldehydes, carboxylates, and esters), -CF₃, -CN and the like. Exemplary substituted alkyls are described below. Cycloalkyls can be further substituted with alkyls, alkenyls, alkoxys, alkylthios, aminoalkyls, carbonyl-substituted alkyls, -CF₃,-CN, and the like.

The term "C_{x-y}" when used in conjunction with a chemical moiety, such as, acyl, acyloxy, alkyl, alkenyl, alkynyl, or alkoxy is meant to include groups that contain from x to y carbons in the chain. For example, the term "C_{x-y}alkyl" refers to substituted or unsubstituted saturated hydrocarbon groups, including straight-chain alkyl and branched-chain alkyl groups that contain from x to y carbons in the chain, including haloalkyl groups such as trifluoromethyl and 2,2,2-tirfluoroethyl, etc. C₀ alkyl indicates a hydrogen where the group is in a terminal position, a bond if internal. The terms "C_{2-y}alkenyl" and "C_{2-y}alkynyl" refer to substituted or unsubstituted unsaturated aliphatic groups analogous in length and possible substitution to the alkyls described above, but that contain at least one double or triple bond respectively.

The term "alkanoyl" refers to a group represented by the general formula hydrocarbyl-C(O)-, preferably alkyl-C(O)-.

The term "alkoxy" (including 'alkoxy' of haloalkoxy) refers to an alkyl group, preferably a lower alkyl group, having an oxygen attached thereto. In some embodiments, preferably, the alkoxy is C₁-C₅ alkoxy, and more preferably C₁-C₃ alkoxy. More specifically, preferable alkoxy includes, but is not limited to, methoxy, ethoxy, and propanoxy. Halogen includes, but is not limited to, fluoride, chloride, bromide, and iodide. Preferably, alkanoyl is C₂-C₁₀ alkanoyl, and more preferably C₃-C₅ alkanoyl. More specifically, preferable alkanoyl includes, but is not limited to, ethanoyl, propanoyl, and cyclohexanecarbonyl.

The terms "amine" and "amino" are art-recognized and refer to both unsubstituted and substituted amines and salts thereof, e.g., a moiety that can be represented by 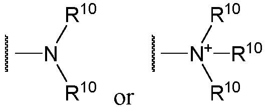 wherein each R¹⁰ independently represents a hydrogen or a hydrocarbyl group, or two R¹⁰ are taken together with the N atom to which they are attached complete a heterocycle having from 4 to 8 atoms in the ring structure.

The term "aryl" as used herein include substituted or unsubstituted single-ring aromatic groups in which each atom of the ring is carbon. Preferably the ring is a 5- to 10-membered ring, more preferably a 6- to 10-membered ring or a 6-membered ring. The term "aryl" also includes polycyclic ring systems having two or more cyclic rings in which two or more carbons are common to two adjoining rings wherein at least one of the rings is aromatic, e.g., the other cyclic rings can be cycloalkyls, cycloalkenyls, cycloalkynyls, aryls, heteroaryls, and/or heterocyclyls. Aryl groups include benzene, naphthalene, phenanthrene, phenol, aniline, and the like. Exemplary substitution on an aryl group can include, for example, a halogen, a haloalkyl such as trifluoromethyl, a hydroxyl, a carbonyl (such as a carboxyl, an alkoxycarbonyl, a formyl, or an acyl such as an alkylC(O)), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a silyl ether, a sulfhydryl, an alkylthio, a sulfate, a sulfonate, a sulfamoyl, a sulfonamido, a sulfonyl, a heterocyclyl, an aralkyl, or an aromatic or heteroaromatic moiety

The terms "halo" and "halogen" as used herein means halogen and includes chloro, fluoro, bromo, and iodo.

The term "lower" when used in conjunction with a chemical moiety, such as, acyl, acyloxy, alkyl, alkenyl, alkynyl, or alkoxy is meant to include groups where there are ten or fewer non-hydrogen atoms in the substituent, preferably six or fewer. A "lower alkyl", for example, refers to an alkyl group that contains ten or fewer carbon atoms, preferably six or fewer. In certain embodiments, acyl, acyloxy, alkyl, alkenyl, alkynyl, or alkoxy substituents defined herein are respectively lower acyl, lower acyloxy, lower alkyl, lower alkenyl, lower alkynyl, or lower alkoxy, whether they appear alone or in combination with other substituents, such as in the recitations hydroxyalkyl and aralkyl (in which case, for example, the atoms within the aryl group are not counted when counting the carbon atoms in the alkyl substituent).

The term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms. Substituents can include any substituents described herein, for example, a halogen, a haloalkyl, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that substituents can themselves be substituted, if appropriate. Unless specifically stated as "unsubstituted," references to chemical moieties herein are understood to include substituted variants. For example, reference to an "aryl" group or moiety implicitly includes both substituted and unsubstituted variants.

The term "pharmaceutically acceptable salt" of the present disclosure means salts produced by non-toxic or little toxic acid or base. In case that the compound of the present disclosure is acidic, base addition salts of the compound of the present disclosure can be made by reacting the free base of the compound with enough amount of desirable base and adequate inert solvent. Pharmaceutically acceptable base addition salt includes, but is not limited to, sodium, potassium, calcium, ammonium, magnesium or salt made by organic amino. In case that the compound of the present disclosure is basic, acid addition salts of the compound of the compound can be made by reacting the free base of the compound with enough amount of desirable acid and adequate inert solvent. Pharmaceutically acceptable acid addition salt includes, but is not limited to, propionic acid, isobutylic acid, oxalic acid, malic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, hydrochloric acid, bromic acid, nitric acid, carbonic acid, monohydrogen-carbonic acid, phosphoric acid, monohydrogen-phosphoric acid, dihydrogen-phosphoric acid, sulfuric acid, monohydrogen-sulfuric acid, hydrogen iodide, and phosphorous acid. In addition, the pharmaceutically acceptable salt of the present disclosure includes, but is not limited to, a salt of amino acid like arginate and an analog of organic acid like glucuronic or galactunoric.

For example, a pharmaceutically acceptable salt of 2-hydroxy-5-[2-(4-trifluoromethyl-phenyl)-ethylamino]-benzoic acid (Compound 2), one preferable example of the present disclosure, can be prepared by the below reaction scheme 1. However, the following reaction methods are offered by way of illustration and are not intended to limit the scope of the disclosure.

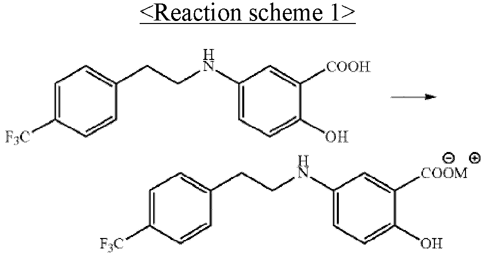

In Scheme 1, M is a pharmaceutically acceptable metal or basic organic compound such as diethylamine, lithium, sodium and potassium.

In more detail, diethylamine salt can be prepared by dissolving a compound in alcohol, adding dropwise diethylamine, stirring the mixture, distilling in vacuo, and crystallizing the residue by adding ether. Alkali metal salt can be made by preparing desirable salt with inorganic reagent like lithium hydroxide, sodium hydroxide, potassium hydroxide in solvent like alcohol, acetone, acetonitrile and then freeze-drying. In addition, according to the similar method, lithium salt can be made with lithium acetate, sodium salt can be made with sodium 2-ethylhexanoate or sodium acetate, and potassium salt can be made with potassium acetate.

Some of the compounds of the present disclosure may be hydrated form and may exist as solvated or unsolvated form. A part of compounds according to the present disclosure exist as crystal form or amorphous form, and any physical form is included in the scope of the present disclosure. In addition, some compounds of the present disclosure may contain one or more asymmetric carbon atoms or double bond, and therefore exists in two or more stereoisomeric forms like racemate, enantiomer, diastereomer, geometric isomer, etc. The present disclosure includes these individual stereoisomers of the compounds.

### Compositions

The present disclosure also provides a composition comprising the 5-benzylaminosalicylic acid derivative represented by the above chemical formula (I) or its pharmaceutically acceptable salt; and pharmaceutically acceptable excipient or additive. The 5-benzylaminosalicylic acid derivative represented by the above chemical formula (I) or its pharmaceutically acceptable salt of the present disclosure may be administered alone. In some embodiments, the composition comprising a compound of formula (I) is administered with any convenient carrier, diluent, etc.

In some embodiments, the composition comprises from about 1 mg to about 1,000 mg of the compound of formula (I). In some embodiments, the composition comprises from about 10 mg to about 1,000 mg of the compound of formula (I). In some embodiments, the composition comprises from about 1 mg to about 500 mg of the compound of formula (I). In some embodiments, the composition comprises from about 1 mg to about 100 mg of the compound of formula (I). In some embodiments, the composition comprises from about 2 mg to about 50 mg of the compound of formula (I).

In some embodiments, a formulation for administration may be single-dose unit or multiple-dose unit. In some embodiments, the composition comprises a single dose unit. In some embodiments, the composition comprises a multiple-dose unit.

The composition for oral administration of the present disclosure may be formulated in a solid or liquid form. The solid formulation includes, but is not limited to, a powder, a granule, a tablet, a capsule, a suppository, etc. Also, the solid formulation may further include, but is not limited to, a diluent, a flavoring agent, a binder, a preservative, a disintegrating agent, a lubricant, a filler, a plasticizer, etc. The liquid formulation includes, but is not limited to, a solution such as water solution and propylene glycol solution, a suspension, an emulsion, etc., and may be prepared by adding suitable additives such as a coloring agent, a flavoring agent, a stabilizer, a thickener, etc. In some embodiments, the composition is administered in a form selected from a capsule, a tablet, a powder, and a solution. In some embodiments, the composition is administered by mixing with a dietary supplement. In some embodiments, the composition is administered as a dietary supplement. In some embodiments, the composition is administered by mixing with food. In some embodiments, the composition is administered as a food composition. In some embodiments, the composition is administered by dissolving in water. In some embodiments, the composition is administered as a capsule with water. In some embodiments, the composition is administered as a chewable tablet.

For example, a powder can be made by simply mixing the 5-benzylaminosalicylic acid derivative of the present disclosure and pharmaceutically acceptable excipients like lactose, starch, microcrystalline cellulose. A granule can be prepared as follows: mixing the compound, a pharmaceutically acceptable excipient. In some embodiments, the pharmaceutically acceptable excipient comprises a diluent and/or a pharmaceutically acceptable binder. In some embodiments, the binder is polyvinylpyrrolidone, hydroxypropylcellulose, etc.

In some embodiments, the composition is formed by wet-granulating with adequate solvent like water, ethanol, isopropanol, etc. In some embodiments, the composition is formed by direct-compressing with compressing power. In addition, a tablet can be made by mixing the granule with a pharmaceutically acceptable lubricant such as magnesium stearate and tableting the mixture.

The pharmaceutical composition of the present disclosure may be administered in forms of, but not limited to, oral formulation, injectable formulation (for example, intramuscular, intraperitoneal, intravenous, infusion, subcutaneous, implant), inhalable, intranasal, vaginal, rectal, sublingual, transdermal, topical, etc. depending on the disorders to be treated and the animal's conditions. The composition of the present disclosure may be formulated in a suitable dosage unit comprising a pharmaceutically acceptable and non-toxic carrier, additive and/ or vehicle, which all are generally used in the art, depending on the routes to be administered. Depot type of formulation being able to continuously release drug for desirable time also is included in the scope of the present disclosure.

In some embodiments, the composition is a capsule comprising:
about 1 mg to about 1000 mg of the compound of formula (I);
about 50% w/w to about 70% w/w lactose monohydrate;
about 2% w/w to about 8% w/w croscarmellose sodium;
about 0.1% w/w to about 1% w/w magnesium stearate; and
about 0.1% w/w to about 2% w/w sodium lauryl sulfate.

In some embodiments, the composition is a capsule comprising:
about 1 mg to about 1000 mg of the compound of formula (I);
about 60% w/w lactose monohydrate;
about 5% w/w croscarmellose sodium;
about 0.5% w/w magnesium stearate; and
about 1% w/w sodium lauryl sulfate.

In some embodiments, the composition is a food composition comprising:
about 1 mg to about 1000 mg of the compound of formula (I);
about 30% w/w to about 50% w/w starch;
about 15% w/w to about 25% w/w crude protein;
about 10% w/w to about 20% crude fat;
about 0.1% w/w to about 5% w/w crude fiber;
about 1% w/w to about 10% w/w crude ash;
about 0.1% w/w to about 5% w/w arginine;
about 0.1% w/w to about 2.5% w/w calcium;
about 0.1% w/w to about 3% w/w lysine;
about 0.1% w/w to about 3% w/w methionine plus cystine; and
about 0.1% w/w to about 2.5% w/w phosphorus.

In some embodiments, the composition is a food composition comprising:
about 1 mg to about 1000 mg of the compound of formula (I);
about 42.7% w/w starch;
about 21.0% w/w crude protein;
about 14% w/w crude fat;
about 1.9% w/w crude fiber;
about 6.1% w/w crude ash;
about 1.4% w/w arginine;
about 0.75% w/w calcium;
about 1.1% w/w lysine;
about 1.18% w/w methionine plus cystine; and
about 0.5% w/w phosphorus.

In some embodiments, the composition is a dietary supplement comprising:
about 1 mg to about 1000 mg of the compound of formula (I);
about 5% w/w to about 20% w/w crude protein;
about 0.1% w/w to about 5% w/w crude fat;
about 0.1% w/w to about 5% w/w crude fiber;
about 0.1% w/w to about 5% w/w crude ash;
about 0% w/w to about 1% w/w calcium;
about 0% w/w to about 2% w/w potassium; and
about 60% w/w to about 95% w/w water.

In some embodiments, the composition is a dietary supplement comprising:
about 1 mg to about 1000 mg of the compound of formula (I);
about 12.0% w/w crude protein;
about 1.5% w/w crude fat;
about 0.4% w/w crude fiber;
about 1.5% w/w crude ash;
about 0.02% w/w calcium;
about 0.1% w/w potassium; and
about 78.0% w/w water.

In some embodiments, the composition is a chewable tablet comprising:
about 1 mg to about 1000 mg of the compound of formula (I);
about 0.1% w/w to about 5% w/w silicon dioxide;
about 0% w/w to about 2% w/w benzoic acid;
about 0% w/w to about 1% w/w sorbic acid;
about 0.1% w/w to about 10% w/w magnesium stearate;
about 10% w/w to about 30% w/w cellulose;
about 30% w/w to about 50% w/w chicken source;
about 0.1% w/w to about 5% w/w dry yeast; and
about 10% w/w to about 30% w/w glucose.

In some embodiments, the composition is a chewable tablet comprising:
about 1 mg to about 1000 mg of the compound of formula (I);
about 3% w/w silicon dioxide;
about 0.05% w/w benzoic acid;
about 0.01% w/w sorbic acid;
about 5% w/w magnesium stearate;
about 20% w/w cellulose;
about 40% w/w chicken powder;
about 3% w/w dry yeast; and
about 19% w/w glucose.

For treating cognitive decline in feline companion animal, the compound of the present disclosure may be administered daily at a dose of approximately 0.01 mg/kg to approximately 200 mg/kg, preferably approximately 0.1 mg/kg to approximately 30 mg/kg. In some embodiments, the compound of formula (I) is administered in a dose from about 0.1 mg per kilogram of body weight to about 10 mg per kilogram of body weight. However, the dosage may be varied according to the animal's conditions (age, sex, body weight, etc.), the severity of disease in animals in need thereof, the used effective components, diets, etc. The compound of the present disclosure may be administered once a day or several times a day in divided doses, if necessary. In some embodiments, the compound of formula (I) is administered one, two, or three times daily. In some embodiments, the compound of formula (I) is administered once daily.

### EXAMPLES

Compound 2, 2-hydroxy-5-[2-(4-trifluoromethyl-phenyl)ethylamino]benzoic acid, was used as a representative compound. Hereinafter, the present disclosure is described in considerable detail to help those skilled in the art understand the present disclosure. However, the following examples are offered by way of illustration and are not intended to limit the scope of the disclosure. The invention is as defined in the claims.

### 1. Study design and subjects

In preliminary data, the maximally beneficial effects such as improvement in cognitive and motor functions were shown in animal models of AD (APP/PS1 mice) and amyotrophic lateral sclerosis (G93A mice) following oral administration of Compound 2 at the dose of 2.5 mg/kg.

Following the oral administration of Compound 2 at the dose of 2.5 mg/kg in mice, Cₘₐₓ (mean maximum plasma concentration) and AUC (area under the plasma level-time curve) were measured 5.19 ± 0.96 µg/mL and 9.61 ± 1.38 µg·hr/mL, respectively.

The systemic toxic potential of Compound 2 in beagle dogs was investigated in Huntington Life Science, a large non-clinical contract research organization in Cambridgeshire, England. Three groups each comprising three males and three females, received Compound 2 at doses of 20, 65, or 200 mg/kg/day for 13 weeks. Daily oral administration of Compound 2 to beagle dogs caused adaptive changes in the liver and kidney and secondary finding in the thyroid. There was no evidence of toxicity, though treated animals tended to struggle at the time of dose administration. The principal treatment-related findings all showed at least partial recovery during the four-week recovery period, with most showing full recovery. Consequently, 200 mg/kg/day was considered the no-observed-adverse-effect level (NOAEL) in dogs.

In pharmacokinetic study, Cₘₐₓ and AUC in beagle dogs were 10.6 µg/mL and 75.1 µg·hr/mL, respectively, following oral administration of 20 mg/kg/day Compound 2. Based upon the safety and pharmacokinetic profile of Compound 2, a dose of 10 mg/kg/day was chosen to investigate safety and efficacy of Compound 2 in canine CDS.

In stability test, Compound 2 was stable at 25 °C for at least 60 months. Additionally, the capsule and chewable formulations of Compound 2 were stable at 25 °C for at least 24 and 12 months, respectively.

The companion animals tested in the study were 22 elderly dogs (12-19 years of age) diagnosed with CDS. The subjects of the examples are presented in Table 1. Each dog enrolled for the present study met the following criteria: body weight <12 kg (regardless of gender), dogs which had lived with the owner for at least 90 days, dogs which were able to take oral medicine, dogs with CDS diagnosed by investigator (canine cognitive dysfunction rating (CCDR) scale ≥ 50 points) and dogs with informed consent of their owners. Dogs with a pregnancy or lactating, with hypersensitivity against salicylic acid derivatives, or with the underlying disease (i.e. renal dysfunction, visual loss, heart failure, or renal failure) were excluded. In addition, dogs which had participated any other clinical trials within 90 days or dogs which had not only CDS but also other neurodegenerative diseases were excluded.

This study was a randomized, blind, placebo-controlled clinical trial to investigate the efficacy and safety of Compound 2 in dogs with CDS. To investigate the efficacy of Compound 2, two questionnaires were evaluated: CCDR scale and canine dementia scale (CADES). To measure the safety of Compound 2, not only the occurrence of adverse events was investigated but also the vital signs, physical examination, and blood tests were performed in the dogs. Compound 2 was prescribed for oral administration of 10 mg/kg once-daily dosing for 8 weeks. Subjects 3 and 4 received Compound 2 additionally for 4 weeks with approval of the principal investigator. Instead of Compound 2, subjects 18-22 received placebo for 8 weeks. In this study, we allowed the dogs to be administered Compound 2 in various ways: mix with dietary supplement (subjects 1 and 3), mix with food (subjects 2 and 6), dissolve in water (subject 4), or capsule with water (subject 5), or chewable tablet with water (subjects 7-22).

**Table 1. Subjects of Examples**

| Subject No. | Dog Name | Breed | Sex | Age | Wt (kgs) |
|---|---|---|---|---|---|
| 1 | Rich | Poodle | M | 15 | 1.9 |
| 2 | Sarang | Mix | F | 18 | 6.7 |
| 3 | Ggandol | Miniature Pinscher | M | 15 | 4.6 |
| 4 | Ddol | Yorkshire terrier | F | 13 | 1.5 |
| 5 | Wanggun | Shih Tzu | M | 14 | 7.1 |
| 6 | Taro | Mix | F | 18 | 3.8 |
| 7 | Donggun | Mixed | M | 12 | 5.7 |
| 8 | Sarang II | Schnauzer | F | 16 | 5.0 |
| 9 | Dream | Schnauzer | F | 15 | 3.6 |
| 10 | Goldstar | Maltese | F | 15 | 2.4 |
| 11 | Fullmoon | Schnauzer | F | 15 | 5.2 |
| 12 | Youngsik | Dachshund | M | 19 | 5.1 |
| 13 | Genie | Toy Poodle | F | 15 | 2.8 |
| 14 | Danvi | Schnauzer | F | 14 | 10.5 |
| 15 | Cherry | Poodle | M | 15 | 3.5 |
| 16 | Wangbok | Maltese | M | 18 | 4.4 |
| 17 | Jorong | Maltese | M | 16 | 4.0 |
| 18 | Sorong | Schnauzer | F | 15 | 3.8 |
| 19 | Wooju | Chihuahua | M | 12 | 3.5 |
| 20 | Luna | Yorkshire terrier | F | 15 | 2.8 |
| 21 | Choco | Cocker Spaniel | M | 13 | 10.5 |
| 22 | Ming | Yorkshire terrier | F | 16 | 3.1 |

### 2. Questionnaires in dogs with CDS

Rating scales are essential tools for CDS diagnosis, staging, assessment, and careful monitoring of the disease symptoms as well as for evaluation of the efficacy of therapeutic strategies. Several rating scales have been developed during the last decade e.g. CCDR scale [21-24]. To assess the severity of CDS in this study, two questionnaires (CCDR scale and CADES) were performed before and after oral administration of Compound 2 by a veterinarian. Questionnaires include a broad range of items measuring appetite, drinking behavior, barking, elimination behavior, day/night sleeping pattern, aimless behavior, adaptive capabilities, social behavior, perceptual ability, disorientation, and memory.

### 2.1 The examination according to CCDR scale

CCDR scale consists of 13 items based on CDS symptoms (Table 2). The 13 kinds of behaviors included various problems related to orientation (staring blankly, getting lost in the home), memory (lack of recognition of owners, house-soiling), apathy (reduced time spent active, avoiding contact with owners), impaired olfaction (difficulty finding food), and locomotion. These problems compromise both the dog's quality of life and the dog-owner bond [25, 26]. Scores ≥50 on the CCDR are indicative of CDS in the aged dogs (12-19 years of age). In this study, we compared the CCDR scores before and after administration of Compound 2 in dogs with CDS.

**Table 2. Canine cognitive dysfunction rating (CCDR) scale**

| | **(1)** | **(2)** | **(3)** | **(4)** | **(5)** | |
|---|---|---|---|---|---|---|
| | **Never** | **Once a month** | **Once a week** | **Once a day** | **>once a day** | **Score** |
| How often does your dog pace up and down, walk in circles and/or wander with no direction or purpose? | | | | | | |
| How often does your dog stare blankly at the walls or floor? | | | | | | |
| How often does your dog get stuck behind objects and is unable to get around? | | | | | | |
| How often does your dog fail to recognize familiar people or pets? | | | | | | |
| How often does your dog walk into walls or doors? How often does your dog walk away while, or avoid, being patted? | | | | | | |
| | **Much less** | **Slightly less** | **The same** | **Slightly more** | **Much more** | |
| Compared with 6 months ago, does your dog now pace up and down, walk in circles and/or wander with no direction or purpose | | | | | | |
| Compared with 6 months ago, does your dog now stare blankly at the walls or floor | | | | | | |
| Compared with 6 months ago, does your dog urinate or defecate in an area it has previously kept clean (if your dog has never house-soiled, tick 'the same') | | | | | | |
| Compared with 6 months ago, does your dog have difficulty finding food dropped on the floor | | | | | | x2 |
| Compare with 6 months ago, does your dog fail to recognize familiar people or pets | | | | | | x3 |

| | **Much more** | **Slightly more** | **The same** | **Slightly less** | **Much less** | |
|---|---|---|---|---|---|---|
| Compared with 6 months ago, is the amount of time your dog spends active | | | | | | |

Although a total of 22 companion dogs with CDS were initiated in the study, 17 companion dogs received 8-week treatment with Compound 2, and subjects 1 and 2 underwent aging-dependent nephrotoxicity and natural death at 7 weeks after administration. Even four weeks after oral administration of Compound 2, most companion dogs with CDS showed the remarkably reduced tendencies for behaviors on the CCDR scale, indicating that dogs treated with Compound 2 showed near normal cognitive function within 4 weeks (Table 3). Also, the frequency of abnormal behaviors was decreased, and their social interaction was improved in most of the dogs. The improved CCDR score after administration of Compound 2 was observed up to 8 weeks.

**Table 3. CCDR Scores of subjects following administration of Compound 2**

| Subjects No. | 0 week | 4 weeks | | 8 weeks | |
|---|---|---|---|---|---|
| | Score | score | change | score | change |
| 1 | 69 | 53 | -16 | - | - |
| 2 | 59 | 49 | -10 | - | - |
| 3 | 74 | 30 | -44 | 30 | -44 |
| 4 | 62 | 49 | -13 | 42 | -20 |
| 5 | 60 | 38 | -22 | 34 | -26 |
| 6 | 51 | 40 | -11 | 46 | -5 |
| 7 | 64 | 55 | -9 | 41 | -23 |
| 8 | 67 | 56 | -11 | 51 | -16 |
| 9 | 65 | 53 | -12 | 54 | -11 |
| 10 | 67 | 55 | -12 | 27 | -40 |
| 11 | 74 | 56 | -18 | 50 | -24 |
| 12 | 59 | 54 | -5 | 53 | -6 |
| 13 | 61 | 60 | -1 | 59 | -2 |
| 14 | 63 | 52 | -11 | 53 | -10 |
| 15 | 59 | 50 | -9 | 50 | -9 |
| 16 | 50 | 43 | -7 | 43 | -7 |
| 17 | 69 | 63 | -6 | 50 | -19 |

To determine whether the beneficial effects of Compound 2 could be maintained after discontinuation of the drug treatment, follow-up studies were performed in 6 companion dogs. In subjects 3, 4, 7, 8, 10, and 11 that received 8- or 12-week treatment with Compound 2, the beneficial effects of Compound 2 were observed over the 4 or 8 weeks after the last administration of the drug (Tables 4 and 5). Importantly, the social interaction, appropriate elimination, and changes in the sleep-wake activity cycle were remarkably improved. This implies that Compound 2 can be applied to treat CDS by improving cognitive deficits and slowing down progression of the disease.

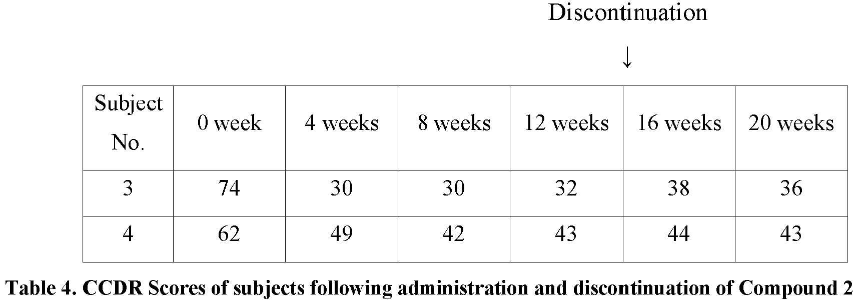

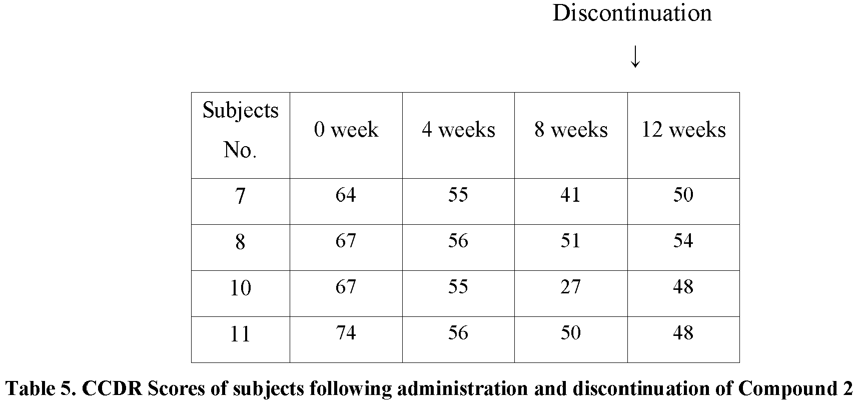

### 2.2 The examination according to CADES

To ensure and confirm the CCDR scale, we used an additional questionnaire, CADES which contains 17 items distributed into four domains, related to changes in dogs' behaviors: spatial orientation, social interaction, sleep-wake cycle, and house soiling (Table 6) [3]. It can be classified according to various stages of cognitive impairment: mild, moderate, and severe cognitive impairment. It is known well that CADES is also suitable for long-term assessment of the progression of cognitive impairment in canine, and potentially as efficacy readout for treatments.

**Table 6. Canine dementia scale (CADES)**

| **Domain/items** | **Score** |
|---|---|
| **A. Spatial orientation** | |
| 1. disorientation in a familiar environment (inside/outside) | |
| 2. to recognize familiar people and animals inside or outside the house/apartment | |
| 3. abnormally respond to familiar objects (a chair, a wastebasket) | |
| 4. aimlessly wandering (motorically restless during day) | |
| 5. a reduced ability to do previously learned task | |
| SCORE (0-25) | |

| **B. Social interaction** | |
|---|---|
| 6. changes in interaction a man/dog, dog/other dog (playing, petting, welcoming) | |
| 7. changes in individual behavior of dog (exploration behavior, play, performance) | |
| 8. response to commands and ability to learn new task | |
| 9. irritable | |
| 10. expression of aggression | |
| SCORE (0-25) | |

| **C. Sleep-wake cycles** | |
|---|---|
| 11. abnormally responds in night (wandering, vocalization, motorically restless) | |
| 12. switch over from insomnia to hypersomnia | |
| SCORE x2 (0-20) | |

| **D. House soiling** | |
|---|---|
| 13. eliminate at home at random locations | |
| 14. eliminate in its kennel or sleeping area | |
| 15. changes in signalization for elimination activity | |
| 16. eliminate indoors after a recent walk outside | |
| 17. eliminate at uncommon locations (grass, concrete) | |
| SCORE (0-25) | |
| **Frequency:** | 0 points - abnormal behavior of the dog was never observed |
| | 2 points - abnormal behavior of the dog was detected at least once in the last 6 months |
| | 3 points - abnormal behavior appeared at least once per month |
| | 4 points - abnormal behavior was seen 2-4 times per month |
| | 5 points - abnormal behavior was observed several times a week. |
| **Total score (A + B + C + D) (0-95)** Clinical stage: Normal aging (Score 0-7), Mild cognitive impairment (8-23), Moderate cognitive impairment (24-44), Severe cognitive impairment (45-95) | |

The scores on spatial orientation and variation were presented in Table 7. At 4 weeks after administration, 10 (59%) of 17 companion dogs showed improvement in their spatial disorientation. Subjects 3, 4, and 10 showed almost normal behavior in spatial orientation items. In 9 (60%) of 15 companion dogs, the beneficial effects were observed at 8 weeks after oral administration of Compound 2. Overall, these results indicate that Compound 2 improves the spatial-oriented ability in dogs with CDS.

**Table 7. CADES scores of spatial orientation (items 1-5) in subjects at 0, 4, and 8 weeks following administration of Compound 2**

| Subjects No. | 0 week score | 4 weeks | | 8 weeks | |
|---|---|---|---|---|---|
| | | score | change | score | change |
| 1 | 24 | 21 | -3 | - | - |
| 2 | 25 | 24 | -1 | - | - |
| 3 | 24 | 5 | -19 | 4 | -20 |
| 4 | 14 | 5 | -9 | 9 | -5 |
| 5 | 23 | 20 | -3 | 15 | -8 |
| 6 | 19 | 18 | -1 | 13 | -6 |
| 7 | 18 | 16 | -2 | 15 | -3 |
| 8 | 18 | 17 | -1 | 17 | -1 |
| 9 | 22 | 24 | 2 | 25 | 3 |
| 10 | 16 | 5 | -11 | 0 | -16 |
| 11 | 24 | 24 | 0 | 24 | 0 |
| 12 | 20 | 20 | 0 | 20 | 0 |
| 13 | 25 | 25 | 0 | 22 | -3 |
| 14 | 20 | 20 | 0 | 20 | 0 |
| 15 | 15 | 13 | -2 | 13 | -2 |
| 16 | 15 | 18 | 3 | 15 | 0 |
| 17 | 20 | 25 | 5 | 23 | 3 |

The social interaction scores in CADES are presented in the Table 8. After 4 weeks administration of Compound 2, social interaction in most participant dogs, except subjects 5, 8, 11, 12, 14, and 17 were markedly improved. Although 3 dogs (subjects 3, 4, and 10) had showed severe abnormal social behaviors before treatment with Compound 2, their social behavior was nearly similar to normal aged dogs at 4 weeks after treatment. In addition, most dogs showed improved social activities at 8 weeks after Compound 2 treatment. More importantly, 8 (53%) of the 15 companion dogs at 8 weeks after administration of Compound 2 showed further improvement in the social interaction compared with improvement at 4 weeks after administration of Compound 2. Overall, these results indicate that Compound 2 improves the social interaction activity in dogs with CDS.

**Table 8. CADES scores of social interaction (items 6-10) in subjects at 0, 4, and 8 weeks following administration of Compound 2**

| Subjects No. | 0 week | 4 weeks | | 8 weeks | |
|---|---|---|---|---|---|
| | score | score | change | score | change |
| 1 | 20 | 19 | -1 | - | - |
| 2 | 20 | 13 | -7 | - | - |
| 3 | 16 | 5 | -11 | 3 | -13 |
| 4 | 15 | 5 | -10 | 15 | 0 |
| 5 | 14 | 14 | 0 | 5 | -9 |
| 6 | 18 | 17 | -1 | 10 | -8 |
| 7 | 19 | 17 | -2 | 14 | -5 |
| 8 | 13 | 13 | 0 | 11 | -2 |
| 9 | 18 | 14 | -4 | 19 | 1 |
| 10 | 14 | 0 | -14 | 4 | -10 |
| 11 | 25 | 25 | 0 | 24 | -1 |
| 12 | 15 | 15 | 0 | 15 | 0 |
| 13 | 19 | 14 | -5 | 16 | -3 |
| 14 | 10 | 10 | 0 | 10 | 0 |
| 15 | 19 | 18 | -1 | 18 | -1 |
| 16 | 15 | 13 | -2 | 9 | -6 |
| 17 | 20 | 20 | 0 | 13 | -7 |

The changes of sleep-wake cycle in CADES were quantified in Table 9. Before administration of Compound 2 all dogs except subject 14 had severe abnormal behaviors on sleep-wake cycle (item 11), but 10 (67%) of 15 companion dogs were significantly improved at 8 weeks after administration of Compound 2. Meanwhile, switch over from insomnia to hypersomnia (item 12) had been observed in 14 (82%) of 17 companion dogs, but the abnormal changes almost disappeared after administration of Compound 2.

**Table 9. CADES scores of sleep-wake cycles (items 11-12) in subjects at 0, 4, and 8 weeks following administration of Compound 2**

| Subjects No. | 0 week score | 4 weeks | | 8 weeks | |
|---|---|---|---|---|---|
| | | score | Change | score | change |
| 1 | 10 | 8 | -2 | - | - |
| 2 | 16 | 10 | -6 | - | - |
| 3 | 16 | 4 | -12 | 0 | -16 |
| 4 | 20 | 18 | -2 | 10 | -10 |
| 5 | 10 | 6 | -4 | 0 | -10 |
| 6 | 18 | 16 | -2 | 14 | -4 |
| 7 | 14 | 18 | 4 | 12 | -2 |
| 8 | 18 | 16 | -2 | 12 | -6 |
| 9 | 20 | 20 | 0 | 20 | 0 |
| 10 | 18 | 0 | -18 | 0 | -18 |
| 11 | 18 | 18 | 0 | 16 | -2 |
| 12 | 10 | 10 | 0 | 0 | -10 |
| 13 | 20 | 20 | 0 | 20 | 0 |
| 14 | 8 | 8 | 0 | 8 | 0 |
| 15 | 20 | 18 | -2 | 16 | -4 |
| 16 | 10 | 14 | 4 | 12 | 2 |
| 17 | 20 | 20 | 0 | 6 | -14 |

The frequency of house soiling was shown in Table 10. After 8 weeks of administration of Compound 2, the house soiling behavior was decreased in all subjects except subjects 4, 12, 13, and 17. Especially, subjects 3, 5, and 10 showed no soiling at 8 weeks after administration of Compound 2.

**Table 10. CADES scores of house soiling (items 13-17) in subjects at 0, 4, and 8 weeks following administration of Compound 2**

| Subjects No. | 0 week | 4 weeks | | 8 weeks | |
|---|---|---|---|---|---|
| | score | score | Change | score | change |
| 1 | 23 | 18 | -5 | - | - |
| 2 | 5 | 4 | -1 | - | - |
| 3 | 10 | 0 | -10 | 0 | -10 |
| 4 | 4 | 5 | 1 | 4 | 0 |
| 5 | 15 | 9 | -6 | 0 | -15 |
| 6 | 18 | 16 | -2 | 8 | -10 |
| 7 | 19 | 14 | -5 | 12 | -7 |
| 8 | 23 | 22 | -1 | 18 | -5 |
| 9 | 15 | 11 | -4 | 14 | -1 |
| 10 | 18 | 3 | -15 | 0 | -18 |
| 11 | 22 | 22 | 0 | 21 | -1 |
| 12 | 19 | 19 | 0 | 20 | 1 |
| 13 | 5 | 5 | 0 | 5 | 0 |
| 14 | 21 | 19 | -2 | 19 | -2 |
| 15 | 20 | 17 | -3 | 17 | -3 |
| 16 | 10 | 10 | 0 | 4 | -6 |
| 17 | 25 | 25 | 0 | 25 | 0 |

Total CADES score was shown in Table 11. The score revealed that the cognitive dysfunction behaviors in all companion dogs with CDS, except subject 9, were gradually decreased after administration of Compound 2. At 8 weeks after oral administration of Compound 2, the significant improvement in cognitive function was observed from severe to moderate, weak, or normal level.

**Table 11. Total CADES Scores of subjects at 0, 4, and 8 weeks following administration of Compound 2**

| Subjects No. | 0 week | 4 weeks | | 8 weeks | |
|---|---|---|---|---|---|
| | score | score | change | Score | change |
| 1 | 77 | 66 | -11 | - | - |
| 2 | 66 | 51 | -15 | - | - |
| 3 | 66 | 14 | -52 | 7 | -59 |
| 4 | 53 | 33 | -20 | 38 | -15 |
| 5 | 62 | 49 | -13 | 20 | -42 |
| 6 | 73 | 67 | -6 | 45 | -28 |
| 7 | 70 | 65 | -5 | 53 | -17 |
| 8 | 72 | 68 | -4 | 58 | -14 |
| 9 | 75 | 69 | -6 | 78 | 3 |
| 10 | 66 | 8 | -58 | 4 | -62 |
| 11 | 89 | 89 | 0 | 85 | -4 |
| 12 | 64 | 64 | 0 | 55 | -9 |
| 13 | 69 | 64 | -5 | 63 | -6 |
| 14 | 59 | 57 | -2 | 57 | -2 |
| 15 | 74 | 66 | -8 | 64 | -10 |
| 16 | 50 | 55 | 5 | 40 | -10 |
| 17 | 85 | 90 | 5 | 67 | -18 |

The beneficial effects of Compound 2 in 6 companion dogs (subjects 3, 4, 7, 8, 10, and 11) were observed even over the next 4 or 8 weeks after the administration was discontinued (Tables 12 and 13). The sleep patterns, house soiling, social interaction, and behavioral activities were significantly improved after treatment with Compound 2. Also, the subjects became more obedient to their owners and showed reduced aggressiveness. These results imply that Compound 2 can be administered to reduce cognitive impairment and slow down disease progression in canine CDS.

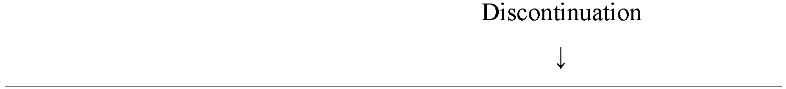

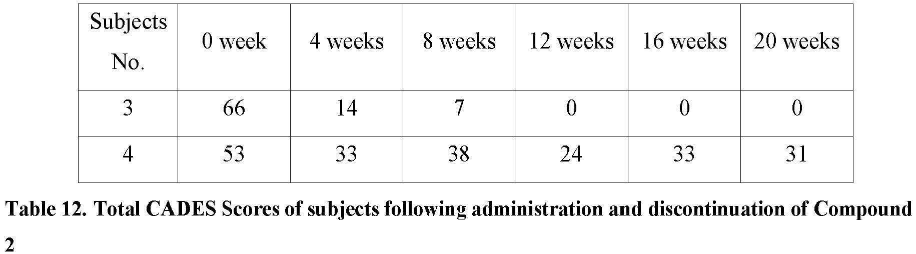

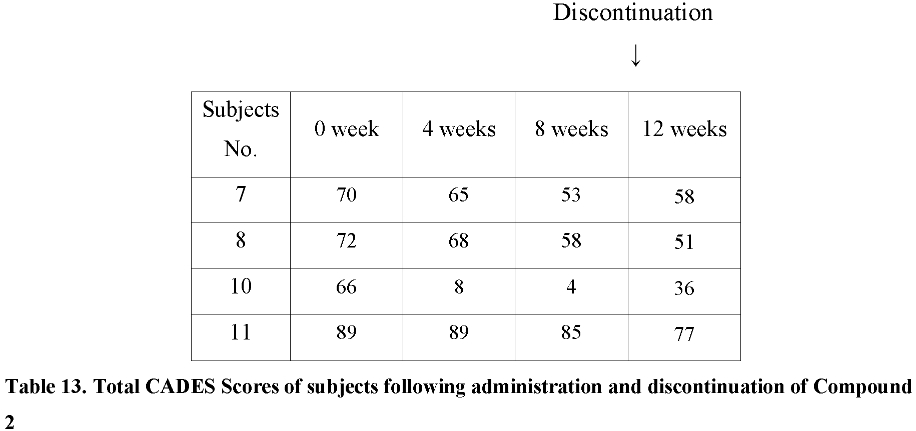

### 2.3 Results of CCDR and CADES in dogs receiving placebo

To clarify the effects of Compound 2 on the cognitive impairment, 5 dogs (subjects 18-22) with CDS received placebo. CCDR and CADES scores in most dogs receiving placebo showed no significant changes or increases at 8 weeks after placebo treatment, indicating that placebo treatment did not affect the cognitive function. Overall, it is strongly implied that Compound 2 can be administered to reduce cognitive impairment in canine CDS.

**Table 14. CCDR Scores of subjects following administration of placebo**

| Subjects No. | 0 week | 4 weeks | | 8 weeks | |
|---|---|---|---|---|---|
| | score | score | change | Score | change |
| 18 | 60 | 60 | 0 | 77 | 17 |
| 19 | 66 | 61 | -5 | 63 | -3 |
| 20 | 58 | 52 | -6 | 52 | -6 |
| 21 | 53 | 64 | 11 | 66 | 13 |
| 22 | 60 | 72 | 12 | 72 | 12 |

**Table 15. Total CADES Scores of subjects following administration of placebo**

| Subjects No. | 0 week | 4 weeks | | 8 weeks | |
|---|---|---|---|---|---|
| | score | score | change | Score | change |
| 18 | 85 | 95 | 10 | 94 | 9 |
| 19 | 73 | 72 | -1 | 82 | 9 |
| 20 | 58 | 52 | -6 | 52 | -6 |
| 21 | 45 | 56 | 11 | 58 | 13 |
| 22 | 79 | 70 | -9 | 93 | 14 |

### 3. Safety assessment in dogs with CDS

During this study, the safety of Compound 2 in dogs with CDS was evaluated at every visit. As a result, no significant changes in the blood toxicity test were observed, and treatment-related adverse events did not occurr as well.

### 4. Conclusion

Two questionnaires were used to evaluate the effects of Compound 2 on cognitive function in the companion dogs with CDS. In the CCDR scale, the administration of Compound 2 substantially improved cognitive function almost back to normal score. Consistent with CCDR scale, severe cognitive and neurobehavioral impairment in dogs with CDS was significantly alleviated within 8 weeks after administration of Compound 2. These beneficial effects were maintained over 4 or 8 weeks after 8- or 12-week administration of Compound 2 was completed. Also, no adverse events or toxicity were observed during the study. On the other hand, there was no significant improvement in placebo group. Taken together, these findings strongly imply that Compound 2 can be applied to treat CDS in canines and also felines.

### INDUSTRIAL APPLICABILITY

The present disclosure provides compositions comprising a compound of formula (I), preferably Compound 2, for use in treating cognitive dysfunction syndrome (CDS) in companion animals, including in canines or felines. The compositions for use in the present disclosure are very useful for reducing or slowing down cognitive and neurobehavioral impairments in the age-related neurological diseases in canines or felines.

### OTHER PUBLICATIONS

1. Azkona, G., et al., Prevalence and risk factors of behavioural changes associated with age-related cognitive impairment in geriatric dogs. J Small Anim Pract, 2009. 50(2): p. 87-91.
2. Neilson, J.C., et al., Prevalence of behavioral changes associated with age-related cognitive impairment in dogs. J Am Vet Med Assoc, 2001. 218(11): p. 1787-91.
3. Madari, A., et al., Assessment of severity and progression of canine cognitive dysfunction syndrome using the CAnine DEmentia Scale (CADES). Appl Anim Behav Sci., 2015. 171: p. 138-45.
4. Salvin, H.E., et al., Under diagnosis of canine cognitive dysfunction: a cross-sectional survey of older companion dogs. The Veterinary Journal, 2010. 184(3): p. 277-281.
5. Hart, B.L. and L.A. Hart, Selecting, raising, and caring for dogs to avoid problem aggression. Journal of the American Veterinary Medical Association, 1997. 210(8): p. 1129.
6. Landsberg, G.M., J. Nichol, and J.A. Araujo, Cognitive dysfunction syndrome: a disease of canine and feline brain aging. Vet Clin North Am Small Anim Pract, 2012. 42(4): p. 749-68, vii.
7. Landsberg, G.M., T. Deporter, and J.A. Araujo, Clinical signs and management of anxiety, sleeplessness, and cognitive dysfunction in the senior pet. Vet Clin North Am Small Anim Pract, 2011. 41(3): p. 565-90.
8. Gonzalez-Martinez, A., et al., Effect of age and severity of cognitive dysfunction on two simple tasks in pet dogs. Vet J, 2013. 198(1): p. 176-81.
9. Cummings, B.J., et al., β-amyloid accumulation correlates with cognitive dysfunction in the aged canine. Neurobiology of learning and memory, 1996. 66(1): p. 11-23.
10. Cummings, B.J., et al., β-amyloid deposition and other measures of neuropathology predict cognitive status in Alzheimer's disease. Neurobiology of aging, 1996. 17(6): p. 921-933.
11. Cummings, B.J., et al., Diffuse plaques contain C-terminal Aβ42 and not Aβ40: evidence from cats and dogs. Neurobiology of aging, 1996. 17(4): p. 653-659.
12. Ozawa, M., et al., The Relation between canine cognitive dysfunction and age-related brain lesions. J Vet Med Sci, 2016. 78(6): p. 997-1006.
13. Rofina, J., et al., Cognitive disturbances in old dogs suffering from the canine counterpart of Alzheimer's disease. Brain research, 2006. 1069(1): p. 216-226.
14. Head, E., et al., Visual-discrimination learning ability and β-amyloid accumulation in the dog. Neurobiology of aging, 1998. 19(5): p. 415-425.
15. Tapp, P.D., et al., Frontal lobe volume, function, and β-amyloid pathology in a canine model of aging. Journal of Neuroscience, 2004. 24(38): p. 8205-8213.
16. Druce, K., Canine cognitive dysfunction-recognition and treatment. Veterinary Nursing Journal, 2014. 29(8): p. 268-270.
17. Ruehl, W., et al., Canine cognitive dysfunction as a model for human age-related cognitive decline, dementia and Alzheimer's disease: clinical presentation, cognitive testing, pathology and response to 1-deprenyl therapy, in Progress in Brain Research. 1995, Elsevier. p. 217-225.
18. Head, E., et al., Spatial learning and memory as a function of age in the dog. Behavioral neuroscience, 1995. 109(5): p. 851.
19. Campbell, S., A. Trettien, and B. Kozan, A non comparative open-label study evaluating the effect of selegiline hydrochloride in a clinical setting. Vet Ther, 2001. 2(1): p. 24-39.
20. Shin, J.H., et al., Concurrent blockade of free radical and microsomal prostaglandin E synthase-1-mediated PGE2 production improves safety and efficacy in a mouse model of amyotrophic lateral sclerosis. Journal of neurochemistry, 2012. 122(5): p. 952-961.
21. Colle, M.A., et al., Vascular and parenchymal Abeta deposition in the aging dog: correlation with behavior. Neurobiol Aging, 2000. 21(5): p. 695-704.
22. Pugliese, M., et al., Severe cognitive impairment correlates with higher cerebrospinal fluid levels of lactate and pyruvate in a canine model of senile dementia. Prog Neuropsychopharmacol Biol Psychiatry, 2005. 29(4): p. 603-10.
23. Schneider, B.M. and M.H. Erhard, [Canine cognitive dysfunction syndrome: prevalence, diagnosis and therapeutic measures]. Tierarztl Prax Ausg K Kleintiere Heimtiere, 2010. 38(2): p. 113-8.
24. Salvin, H.E., et al., The canine cognitive dysfunction rating scale (CCDR): a data-driven and ecologically relevant assessment tool. Vet J, 2011. 188(3): p. 331-6.
25. Hughes, C.P., et al., A new clinical scale for the staging of dementia. Br J Psychiatry, 1982. 140: p. 566-72.
26. Swan, G.E. and D. Carmelli, Impaired olfaction predicts cognitive decline in nondemented older adults. Neuroepidemiology, 2002. 21(2): p. 58-67.

## Claims

1. A composition comprising a compound of formula (I): 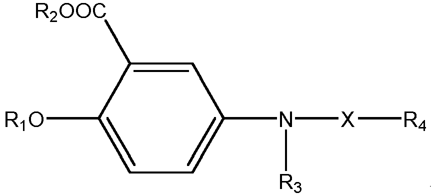 wherein
X is selected from CO, SO₂ and (CH₂)*ₙ*;
R₁ is selected from hydrogen, C₁-C₆ alkyl and C₁-C₆ alkanoyl;
R₂ is selected from hydrogen and C₁-C₆ alkyl;
R₃ is selected from hydrogen and a C₁-C₅ acetyl group; and
R₄ is a phenyl group, which is unsubstituted or substituted with one or more of the group consisting of nitro, halogen, C₁-C₆ alkyl, C₁-C₆ haloalkyl, C₁-C₅ alkoxy, and C₁-C₅ haloalkoxy;
n is an integer from 1 to 5, inclusive;
or a pharmaceutically acceptable salt thereof; and
a pharmaceutically acceptable excipient,
for use in treating cognitive dysfunction syndrome (CDS) in a companion animal.

2. The composition for use according to claim 1, wherein the compound of formula (I) is selected from the group consisting of 2-hydroxy-5-phenethylamino-benzoic acid, 2-hydroxy-5-[2-(4-trifluoromethyl-phenyl)-ethylamino]-benzoic acid, 2-hydroxy-5-[2-(3-trifluoromethyl-phenyl)-ethylamino]-benzoic acid, 5-[2-(3,5-bis-trifluoromethyl-phenyl)-ethylamino]-2-hydroxy-benzoic acid, 2-hydroxy-5-[2-(2-nitro-phenyl)-ethylamino]-benzoic acid, 5-[2-(4-chloro-phenyl)-ethylamino]-2-hydroxy-benzoic acid, 5-[2-(3,4-difluorophenyl)-ethylamino]-2-hydroxy-benzoic acid, 5-[2-(3,4-dichloro-phenyl)-ethylamino]-2-hydroxy-benzoic acid, 5-[2-(4-fluoro-2-trifluoromethyl-phenyl)-ethylamino]-2-hydroxybenzoic acid, 5-[2-(2-fluoro-4-trifluoromethyl-phenyl)-ethylamino]-2-hydroxy-benzoic acid, 2-hydroxy-5-[2-(4-methoxy-phenyl)-ethylamino]-benzoic acid, 2-hydroxy-5-(2-o-tolyl-ethylamino)-benzoic acid, 2-hydroxy-5-(3-phenyl-propylamino)-benzoic acid, 2-hydroxy-5-[3-(4-trifluoromethyl-phenyl)-propylamino]-benzoic acid, 5-[3-(4-fluoro-phenyl)-propylamino]-2-hydroxy-benzoic acid, 5-[3-(3,4-dichloro-phenyl)-propylamino]-2-hydroxybenzoic acid, 2-hydroxy-5-(3-p-tolyl-propylamino)-benzoic acid, 2-acetoxy-5-[2-(4-trifluoromethyl-phenyl)-ethylamino]-benzoic acid, 5-[2-(2-chloro-phenyl)-ethylamino]-2-hydroxy-benzoic acid, 5-benzylaminosalicylic acid, 5-(4-nitrobenzyl)aminosalicylic acid, 5-(4-chlorobenzyl)aminosalicylic acid, 5-(4-trifluoromethylbenzyl)aminosalicylic acid, 5-(4-fluorobenzyl)aminosalicylic acid, 5-(4-methoxybenzyl)aminosalicylic acid, 5-(2,3,4,5,6-pentafluorobenzyl)aminosalicylic acid, 5-(4-nitrobenzyl)amino-2-hydroxy ethylbenzoate, 5-(4-nitrobenzyl)-N-acetylamino-2-hydroxy ethylbenzoate, 5-(4-nitrobenzyl)-N-acetylamino-2-acetoxy ethylbenzoate, 5-(4-nitrobenzoyl)aminosalicylic acid, 5-(4-nitrobenzenesulfonyl)aminosalicylic acid, 5-[2-(4-nitrophenyl)-ethyl]aminosalicylic acid, and 5-[3-(4-nitro-phenyl)-n-propyl]aminosalicylic acid.

3. The composition for use according to claim 2, wherein the compound of formula (I) is 2-hydroxy-5-[2-(4-trifluoromethyl-phenyl)-ethylamino]-benzoic acid.

4. The composition for use according to any one of claims 1-3, which comprises 1 mg to 1000 mg of the compound of formula (I) or its pharmaceutically acceptable salt.

5. The composition for use according to any one of claims 1-4, wherein the composition is suitable for oral administration.

6. The composition for use according to any one of claims 1-5, which is:
(a) a capsule comprising:
1 mg to 1000 mg of the compound of formula (I),
60% w/w lactose monohydrate,
5% w/w croscarmellose sodium,
0.5% w/w magnesium stearate, and
1% w/w sodium lauryl sulfate;
(b) a food composition comprising:
1 mg to 1000 mg of the compound of formula (I),
42.7% w/w starch,
21.0% w/w crude protein,
14% w/w crude fat,
1.9% w/w crude fiber,
6.1% w/w crude ash,
1.4% w/w arginine,
0.75% w/w calcium,
1.1% w/w lysine,
1.18% w/w methionine plus cystine, and
0.5% w/w phosphorus;
(c) a dietary supplement comprising:
1 mg to 1000 mg of the compound of formula (I),
12.0% w/w crude protein,
1.5% w/w crude fat,
0.4% w/w crude fiber,
1.5% w/w crude ash,
0.02% w/w calcium,
0.1% w/w potassium, and
78.0% w/w water; or
(d) a chewable tablet comprising:
1 mg to 1000 mg of the compound of formula (I),
3% w/w silicon dioxide,
0.05% w/w benzoic acid,
0.01% w/w sorbic acid,
5% w/w magnesium stearate,
20% w/w cellulose,
40% w/w chicken source,
3% w/w dry yeast, and
19% w/w glucose.

7. The composition for use according to any one of claims 1-6, wherein of the composition treats CDS through
(a) concurrent pharmacological inhibition of oxidative stress and inflammation;
(b) inhibiting of oxidative stress and prostaglandin E₂ synthesis; or
(c) inhibiting of oxidative stress and microsomal prostaglandin E synthase-1.

8. The composition for use according to claim 1, wherein the composition comprises 2-hydroxy-5-[2-(4-trifluoromethyl-phenyl)-ethylamino]-benzoic acid, which is for administration in a dose from 1 mg/kg of body weight to 200 mg/kg of body weight once a day.

9. The composition for use according to any one of claims 1-8, wherein the companion animal is selected from a cat, a chinchilla, a dog, a ferret, a gerbil, a guinea pig, a hamster, a hedgehog, a mouse, a rabbit, and a rat.

10. The composition for use according to claim 9, wherein the companion animal is a dog or a cat.

11. The composition for use according to claim 1, wherein the companion animal is a feline and the composition comprises 2-hydroxy-5-[2-(4-trifluoromethyl-phenyl)-ethylamino]-benzoic acid, which is for administration in a dose from about 0.1 mg/kg of body weight to about 30 mg/kg of body weight once a day.

12. The composition for use according to claim 1, wherein the composition comprises 2-hydroxy-5-[2-(4-trifluoromethyl-phenyl)-ethylamino]-benzoic acid, which is for administration in a dose from about 0.1 mg/kg of body weight to about 10 mg/kg of body weight once a day.

13. The composition for use according to claim 3, wherein the companion animal is a dog and the treatment improves the cognitive function of the dog at 8 weeks after oral administration.

14. The composition for use according to claim 13, wherein
(a) the composition improves the cognitive function of the dog from severe to moderate, weak, or normal level; or
(b) the composition restores near normal cognitive function to the dog.

15. The composition for use according to claim 13 or 14, wherein the cognitive function is selected from improvement in social interaction, appropriate elimination, spatial orientation, and the sleep-wake activity cycle.

## Patentansprüche

1. Zusammensetzung, umfassend eine Verbindung der Formel (I): 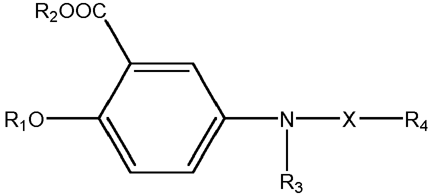 wobei
X ausgewählt ist aus CO, SO₂ und (CH₂)*ₙ*;
R₁ ausgewählt ist aus Wasserstoff, C₁-C₆-Alkyl und C₁-C₆-Alkanoyl;
R₂ ausgewählt ist aus Wasserstoff und C₁-C₆-Alkyl;
R₃ ausgewählt ist aus Wasserstoff und einer C₁-C₅-Acetylgruppe; und
R₄ eine Phenylgruppe ist, die unsubstituiert oder substituiert mit einem oder mehreren aus der Gruppe bestehend aus Nitro, Halogen, C₁-C₆-Alkyl, C₁-C₆-Haloalkyl, C₁-C₅-Alkoxy und C₁-C₅-Haloalkoxy ist;
n eine ganze Zahl von 1 bis einschließlich 5 ist;
oder ein pharmazeutisch verträgliches Salz davon; und
einen pharmazeutisch verträglichen Hilfsstoff,
zur Verwendung beim Behandeln von kognitivem Dysfunktionssyndrom (CDS) bei einem Haustier.

2. Zusammensetzung zur Verwendung nach Anspruch 1, wobei die Verbindung der Formel (I) ausgewählt ist aus der Gruppe bestehend aus 2-Hydroxy-5-phenethylamino-benzoesäure, 2-Hydroxy-5-[2-(4-trifluormethyl-phenyl)-ethylamino]-benzoesäure, 2-Hydroxy-5-[2-(3-trifluormethyl-phenyl)-ethylamino]-benzoesäure, 5-[2-(3,5-bis-Trifluormethyl-phenyl)-ethylamino]-2-hydroxy-benzoesäure, 2-Hydroxy-5-[2-(2-nitro-phenyl)-ethylamino]-benzoesäure, 5-[2-(4-Chlor-phenyl)-ethylamino]-2-hydroxy-benzoesäure, 5-[2-(3,4-Difluor-phenyl)-ethylamino]-2-hydroxy-benzoesäure, 5-[2-(3,4-Dichlor-phenyl)-ethylamino]-2-hydroxybenzoesäure, 5-[2-(4-Fluor-2-trifluormethyl-phenyl)-ethylamino]-2-hydroxy-benzoesäure, 5-[2-(2-Fluor-4-trifluormethyl-phenyl)-ethylamino]-2-hydroxy-benzoesäure, 2-Hydroxy-5-[2-(4-methoxy-phenyl)-ethylamino]-benzoesäure, 2-Hydroxy-5-(2-o-tolyl-ethylamino)-benzoesäure, 2-Hydroxy-5-(3-phenyl-propylamino)-benzoesäure, 2-Hydroxy-5-[3-(4-trifluormethyl-phenyl)-propylamino]-benzoesäure, 5-[3-(4-Fluor-phenyl)-propylamino]-2-hydroxy-benzoesäure, 5-[3-(3,4-Dichlor-phenyl)-propylamino]-2-hydroxy-benzoesäure, 2-Hydroxy-5-(3-p-tolyl-propylamino)-benzoesäure, 2-Acetoxy-5-[2-(4-trifluormethyl-phenyl)-ethylamino]-benzoesäure, 5-[2-(2-Chlor-phenyl)-ethylamino]-2-hydroxy-benzoesäure, 5-Benzylaminosalicylsäure, 5-(4-Nitrobenzyl)aminosalicylsäure, 5-(4-Chlorbenzyl)aminosalicylsäure, 5-(4-Trifluormethylbenzyl)aminosalicylsäure, 5-(4-Fluorbenzyl)aminosalicylsäure, 5-(4-Methoxybenzyl)aminosalicylsäure, 5-(2,3,4,5,6-Pentafluorbenzyl)aminosalicylsäure, 5-(4-Nitrobenzyl)amino-2-hydroxyethylbenzoat, 5-(4-Nitrobenzyl)-N-acetylamino-2-hydroxyethylbenzoat, 5-(4-Nitrobenzyl)-N-acetylamino-2-acetoxyethylbenzoat, 5-(4-Nitrobenzoyl)aminosalicylsäure, 5-(4-Nitrobenzolsulfonyl)aminosalicylsäure, 5-[2-(4-Nitrophenyl)-ethyl]aminosalicylsäure und 5-[3-(4-Nitro-phenyl)-n-propyl]aminosalicylsäure.

3. Zusammensetzung zur Verwendung nach Anspruch 2, wobei die Verbindung der Formel (I) 2-Hydroxy-5-[2-(4-trifluormethyl-phenyl)-ethylamino]-benzoesäure ist.

4. Zusammensetzung zur Verwendung nach einem der Ansprüche 1-3, die 1 mg bis 1000 mg der Verbindung der Formel (I) oder ihres pharmazeutisch verträglichen Salzes umfasst.

5. Zusammensetzung zur Verwendung nach einem der Ansprüche 1-4, wobei die Zusammensetzung zur oralen Verabreichung geeignet ist.

6. Zusammensetzung zur Verwendung nach einem der Ansprüche 1-5, die wie folgt ist:
(a) eine Kapsel, umfassend:
1 mg bis 1000 mg der Verbindung der Formel (I),
60 Gew.-% Lactosemonohydrat,
5 Gew.-% Croscarmellose-Natrium,
0,5 Gew.-% Magnesiumstearat, und
1 Gew.-% Natriumlaurylsulfat;
(b) eine Lebensmittelzusammensetzung, umfassend:
1 mg bis 1000 mg der Verbindung der Formel (I),
42,7 Gew.-% Stärke,
21,0 Gew.-% Rohprotein,
14 Gew.-% Rohfett,
1,9 Gew.-% Rohfaser,
6,1 Gew.-% Rohasche,
1,4 Gew.-% Arginin,
0,75 Gew.-% Calcium,
1,1 Gew.-% Lysin,
1,18 Gew.-% Methionin plus Cystin, und
0,5 Gew.-% Phosphor;
(c) ein Nahrungsergänzungsmittel, umfassend:
1 mg bis 1000 mg der Verbindung der Formel (I),
12,0 Gew.-% Rohprotein,
1,5 Gew.-% Rohfett,
0,4 Gew.-% Rohfaser,
1,5 Gew.-% Rohasche,
0,02 Gew.-% Calcium,
0,1 Gew.-% Kalium, und
78,0 Gew.-% Wasser; oder
(d) eine Kautablette, umfassend:
1 mg bis 1000 mg der Verbindung der Formel (I),
3 Gew.-% Siliziumdioxid,
0,05 Gew.-% Benzoesäure,
0,01 Gew.-% Sorbinsäure,
5 Gew.-% Magnesiumstearat,
20 Gew.-% Cellulose,
40 Gew.-% Hühnerquelle,
3 Gew.-% Trockenhefe, und
19 Gew.-% Glucose.

7. Zusammensetzung zur Verwendung nach einem der Ansprüche 1-6, wobei die Zusammensetzung CDS durch
(a) gleichzeitige pharmakologische Hemmung von oxidativem Stress und Entzündung;
(b) Hemmen von oxidativem Stress und Prostaglandin-E₂-Synthese; oder
(c) Hemmen von oxidativem Stress und mikrosomaler Prostaglandin-E-Synthase-1 behandelt.

8. Zusammensetzung zur Verwendung nach Anspruch 1, wobei die Zusammensetzung 2-Hydroxy-5-[2-(4-trifluormethyl-phenyl)-ethylamino]-benzoesäure umfasst, die zur Verabreichung in einer Dosis von 1 mg/kg Körpergewicht bis 200 mg/kg Körpergewicht einmal am Tag ist.

9. Zusammensetzung zur Verwendung nach einem der Ansprüche 1-8, wobei das Haustier ausgewählt ist aus einer Katze, einem Chinchilla, einem Hund, einem Frettchen, einer Rennmaus, einem Meerschweinchen, einem Hamster, einem Igel, einer Maus, einem Kaninchen und einer Ratte.

10. Zusammensetzung zur Verwendung nach Anspruch 9, wobei das Haustier ein Hund oder eine Katze ist.

11. Zusammensetzung zur Verwendung nach Anspruch 1, wobei das Haustier katzenartig ist und die Zusammensetzung 2-Hydroxy-5-[2-(4-trifluormethyl-phenyl)-ethylamino]-benzoesäure umfasst, die zur Verabreichung in einer Dosis von etwa 0,1 mg/kg Körpergewicht bis etwa 30 mg/kg Körpergewicht einmal am Tag ist.

12. Zusammensetzung zur Verwendung nach Anspruch 1, wobei die Zusammensetzung 2-Hydroxy-5-[2-(4-trifluormethyl-phenyl)-ethylamino]-benzoesäure umfasst, die zur Verabreichung in einer Dosis von etwa 0,1 mg/kg Körpergewicht bis etwa 10 mg/kg Körpergewicht einmal am Tag ist.

13. Zusammensetzung zur Verwendung nach Anspruch 3, wobei das Haustier ein Hund ist und die Behandlung die kognitive Funktion des Hundes 8 Wochen nach oraler Verabreichung verbessert.

14. Zusammensetzung zur Verwendung nach Anspruch 13, wobei
(a) die Zusammensetzung die kognitive Funktion des Hundes von schwer zu moderat, schwach oder Normalniveau verbessert; oder
(b) die Zusammensetzung nahezu normale kognitive Funktion bei dem Hund wiederherstellt.

15. Zusammensetzung zur Verwendung nach Anspruch 13 oder 14, wobei die kognitive Funktion ausgewählt ist aus Verbesserung der sozialen Interaktion, angemessener Eliminierung, räumlicher Orientierung und dem Schlaf-Wach-Aktivitätszyklus.

## Revendications

1. Composition comprenant un composé de formule (I) : 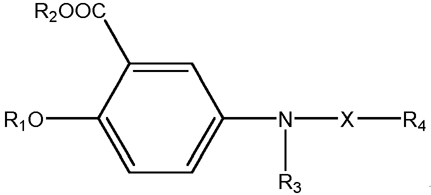 dans laquelle :
X est choisi parmi CO, SO₂ et (CH₂)*ₙ* ;
R₁ est choisi parmi un hydrogène, un alkyle en C₁-C₆ et un alcanoyle en C₁-C₆ ;
R₂ est choisi parmi un hydrogène et un alkyle en C₁-C₆ ;
R₃ est choisi parmi un hydrogène et un groupe acétyle en C₁-C₅ ; et
R₄ est un groupe phényle, qui est non substitué ou substitué par un ou plusieurs éléments du groupe constitué par un nitro, un halogène, un alkyle en C₁-C₆, un halogénoalkyle en C₁-C₆, un alcoxy en C₁-C₅ et un halogénoalcoxy en C₁-C₅ ;
n est un entier compris entre 1 et 5, bornes comprises ;
ou un sel pharmaceutiquement acceptable de celui-ci ; et
un excipient pharmaceutiquement acceptable,
destiné à être utilisé dans le traitement du syndrome de dysfonctionnement cognitif (SDC) chez un animal de compagnie.

2. Composition destinée à être utilisée selon la revendication 1, dans laquelle le composé de formule (I) est choisi dans le groupe constitué par l'acide 2-hydroxy-5-phénéthylamino-benzoïque, l'acide 2-hydroxy-5-[2-(4-trifluorométhyl-phényl)-éthylamino]-benzoïque, l'acide 2-hydroxy-5-[2-(3-trifluorométhyl-phényl)-éthylamino]-benzoïque, l'acide 5-[2-(3,5-bis-trifluorométhyl-phényl)-éthylamino]-2-hydroxy-benzoïque, l'acide 2-hydroxy-5-[2-(2-nitro-phényl)-éthylamino]-benzoïque, l'acide 5-[2-(4-chloro-phényl)-éthylamino]-2-hydroxy-benzoïque, l'acide 5-[2-(3,4-difluoro-phényl)-éthylamino]-2-hydroxy-benzoïque, l'acide 5-[2-(3,4-dichloro-phényl)-éthylamino]-2-hydroxy-benzoïque, l'acide 5-[2-(4-fluoro-2-trifluorométhyl-phényl)-éthylamino]-2-hydroxy-benzoïque, l'acide 5-[2-(2-fluoro-4-trifluorométhyl-phényl)-éthylamino]-2-hydroxy-benzoïque, l'acide 2-hydroxy-5-[2-(4-méthoxy-phényl)-éthylamino]-benzoïque, l'acide 2-hydroxy-5-(2-o-tolyl-éthylamino)-benzoïque, l'acide 2-hydroxy-5-(3-phényl-propylamino)-benzoïque, l'acide 2-hydroxy-5-[3-(4-trifluorométhyl-phényl)-propylamino]-benzoïque, l'acide 5-[3-(4-fluoro-phényl)-propylamino]-2-hydroxy-benzoïque, l'acide 5-[3-(3,4-dichloro-phény])-propylamino]-2-hydroxy-benzoïque, l'acide 2-hydroxy-5-(3-p-tolyl-propylamino)-benzoïque, l'acide 2-acétoxy-5-[2-(4-trifluorométhyl-phényl)-éthylamino]-benzoïque, l'acide 5-[2-(2-chloro-phényl)-éthylamino]-2-hydroxy-benzoïque, l'acide 5-benzylaminosalicylique, l'acide 5-(4-nitrobenzyl)aminosalicylique, l'acide 5-(4-chlorobenzyl)aminosalicylique, l'acide 5-(4-trifluorométhylbenzyl)aminosalicylique, l'acide 5-(4-fluorobenzyl)aminosalicylique, l'acide 5-(4-méthoxybenzyl)aminosalicylique, l'acide 5-(2,3,4,5,6-pentafluorobenzyl)aminosalicylique, le 5-(4-nitrobenzyl)amino-2-hydroxybenzoate d'éthyle, le 5-(4-nitrobenzyl)-N-acétylamino-2-hydroxybenzoate d'éthyle, le 5-(4-nitrobenzyl)-N-acétylamino-2-acétoxybenzoate d'éthyle, l'acide 5-(4-nitrobenzoyl)aminosalicylique, l'acide 5-(4-nitrobenzènesulfonyl)aminosalicylique, l'acide 5-[2-(4-nitrophényl)-éthyl]aminosalicylique et l'acide 5-[3-(4-nitro-phényl)-n-propyl]aminosalicylique.

3. Composition destinée à être utilisée selon la revendication 2, dans laquelle le composé de formule (I) est l'acide 2-hydroxy-5-[2-(4-trifluorométhyl-phényl)-éthylamino]-benzoïque.

4. Composition destinée à être utilisée selon l'une quelconque des revendications 1 à 3, qui comprend 1 mg à 1000 mg du composé de formule (I) ou de son sel pharmaceutiquement acceptable.

5. Composition destinée à être utilisée selon l'une quelconque des revendications 1 à 4, ladite composition étant appropriée pour une administration orale.

6. Composition destinée à être utilisée selon l'une quelconque des revendications 1 à 5, qui est :
(a) une capsule comprenant :
1 mg à 1000 mg du composé de formule (I),
60 % p/p de lactose monohydraté,
5 % p/p de croscarmellose sodique,
0,5 % p/p de stéarate de magnésium, et
1 % p/p de laurylsulfate de sodium ;
(b) une composition alimentaire comprenant :
1 mg à 1000 mg du composé de formule (I),
42,7 % p/p d'amidon,
21,0 % p/p de protéines brutes,
14 % p/p de matières grasses brutes,
1,9 % p/p de fibres brutes,
6,1 % p/p de cendres brutes,
1,4 % p/p d'arginine,
0,75 % p/p de calcium,
1,1 % p/p de lysine,
1,18 % p/p de méthionine plus cystine, et
0,5 % p/p de phosphore ;
(c) un complément alimentaire comprenant :
1 mg à 1000 mg du composé de formule (I),
12,0 % p/p de protéines brutes,
1,5 % p/p de matières grasses brutes,
0,4 % p/p de fibres brutes,
1,5 % p/p de cendres brutes,
0,02 % p/p de calcium,
0,1 % p/p de potassium, et
78,0 % p/p d'eau ; ou
(d) un comprimé à croquer comprenant :
1 mg à 1000 mg du composé de formule (I),
3 % p/p de dioxyde de silicium,
0,05 % p/p d'acide benzoïque,
0,01 % p/p d'acide sorbique,
5 % p/p de stéarate de magnésium,
20 % p/p de cellulose,
40 % p/p de source de poulet,
3 % p/p de levure sèche, et
19 % p/p de glucose.

7. Composition destinée à être utilisée selon l'une quelconque des revendications 1 à 6, ladite composition traitant le SDC par
(a) l'inhibition pharmacologique concomitante du stress oxydatif et de l'inflammation ;
(b) l'inhibition du stress oxydatif et de la synthèse de la prostaglandine E₂ ; ou
(c) l'inhibition du stress oxydatif et de la prostaglandine E synthase-1 microsomale.

8. Composition destinée à être utilisée selon la revendication 1, ladite composition comprenant de l'acide 2-hydroxy-5-[2-(4-trifluorométhyl-phényl)-éthylamino]-benzoïque, qui est destiné à être administré en une dose de 1 mg/kg de poids corporel à 200 mg/kg de poids corporel une fois par jour.

9. Composition destinée à être utilisée selon l'une quelconque des revendications 1 à 8, ledit animal de compagnie étant choisi parmi un chat, un chinchilla, un chien, un furet, une gerbille, un cochon d'Inde, un hamster, un hérisson, une souris, un lapin et un rat.

10. Composition destinée à être utilisée selon la revendication 9, ledit animal de compagnie étant un chien ou un chat.

11. Composition destinée à être utilisée selon la revendication 1, ledit animal de compagnie étant un félin et ladite composition comprenant de l'acide 2-hydroxy-5-[2-(4-trifluorométhyl-phényl)-éthylamino]-benzoïque, qui est destiné à être administré en une dose d'environ 0,1 mg/kg de poids corporel à environ 30 mg/kg de poids corporel une fois par jour.

12. Composition destinée à être utilisée selon la revendication 1, ladite composition comprenant de l'acide 2-hydroxy-5-[2-(4-trifluorométhyl-phényl)-éthylamino]-benzoïque, qui est destiné à être administré en une dose d'environ 0,1 mg/kg de poids corporel à environ 10 mg/kg de poids corporel une fois par jour.

13. Composition destinée à être utilisée selon la revendication 3, ledit animal de compagnie étant un chien et ledit traitement améliorant la fonction cognitive du chien 8 semaines après l'administration orale.

14. Composition destinée à être utilisée selon la revendication 13, dans laquelle
(a) la composition améliore la fonction cognitive du chien de niveau sévère à modéré, faible ou normal ; ou
(b) la composition restaure une fonction cognitive proche de la normale pour le chien.

15. Composition destinée à être utilisée selon l'une des revendications 13 ou 14, ladite fonction cognitive étant choisie parmi l'amélioration de l'interaction sociale, l'élimination appropriée, l'orientation spatiale et le cycle d'activité veille-sommeil.
